(19) Europäisches Patentamt
European Patent Office
Office européen des brevets

(11) Numéro de publication: **0 196 982**
**B1**

(12) **FASCICULE DE BREVET EUROPÉEN**

(45) Date de publication du fascicule du brevet: **06.12.89**

(51) Int. Cl.⁴: **A 61 K 7/155**

(21) Numéro de dépôt: **86420055.5**

(22) Date de dépôt: **20.02.86**

(54) **Produit épilatoire comportant une composition organopolysiloxanique réticulable à température ambiante en un élastomère silicone et procédé d'épilation utilisant ledit produit.**

(30) Priorité: **05.03.85 FR 8503178**

(43) Date de publication de la demande: **08.10.86 Bulletin 86/41**

(45) Mention de la délivrance du brevet: **06.12.89 Bulletin 89/49**

(84) Etats contractants désignés: **AT BE CH DE FR GB IT LI LU NL SE**

(56) Documents cité:
**FR-A-2 204 398**
**GB-A-2 079 762**
**US-A-4 282 877**

(73) Titulaire: **RHONE- POULENC CHIMIE, 25, quai Paul Doumer, F-92408 Courbevoie Cédex (FR)**

(72) Inventeur: **Cyprien, Guy, 7, rue d'Anjou, F-94240 L'Hay Les Roses (FR)**

(74) Mandataire: **Seugnet, Jean Louis, RHONE- POULENC INTERSERVICES Service Brevets Chimie Centre de Recherches de Saint- Fons B.P. 62, F-69192 Saint- Fons Cédex (FR)**

**EP 0 196 982 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

La présente invention concerne un procédé d'épilation au moyen d'une composition organosiloxane réticulable à température ambiante en un élastomère silicone.

On connaît trois types de procédés permettant de supprimer les poils et toute pilosité situées sur les zones de peau où leur présence est considérée comme inopportune généralement pour des raisons esthétiques.

Le premier type de procédé est le simple rasage qui présente l'avantage d'être rapide et indolore mais qui présente l'inconvénient majeur de ne pas aboutir au but visé. En effet, même immédiatement après le rasage, la racine des poils affleurant la peau est toujours visible et, par ailleurs, les poils se trouvent renforcés par le rasage et repoussent avec un aspect plus raide et avec une plus grande longueur qu'avant le rasage.

Le deuxième type de procédé consiste à utiliser un produit cosmétologique dépilatoire se présentant sous la forme d'un liquide ou d'une crème à étaler sur les zones à dépiler. Le produit qui contient comme agent actif un thioglycolate ou un thiolactate de calcium ou de strontium dissout le poil.

Ce procédé est certes indolore et efficace mais la racine du poil subsistant, le poil repousse et quelquefois à l'intérieur de la peau nécessitant des traitement successifs rapprochés. Ces nombreux traitements peuvent à la longue susciter des phénomènes d'allergie et/ou d'irritation.

Le troisième type de procédé consiste à épiler, c'est à dire à arracher les poils à la pince à épiler procédé long et fastidieux ou bien pour effectuer une épilation rapide par le procédé d'enduction à la cire froide, ou chaude, éventuellement appliquée à l'aide de supports souples.

Ce type de procédé est rapide et efficace car la racine du poil étant arrachée les poils ne repoussent qu'au bout de plusieurs mois.

Cependant ce procédé, dans le cas où il fait intervenir un chauffage de la cire, bien qu'ayant l'avantage d'être moins douloureux car la chaleur ramollit le poil, présente l'inconvénient de requérir une installation onéreuse, difficilement transportable. Par ailleurs, la chaleur appliquée à la peau peut être la cause d'irritations et de brûlures allant même jusqu'à l'apparition de varices.

Enfin, les procédés tels que décrits dans le brevet français FR-A-2 500 282 et américain US-A-4 282 877 utilisant la cire ou une pellicule en matière auto-adhésive par pression sur un support souple ne permettent pas d'aboutir à un résultat satisfaisant. En effet, l'épilation est généralement douloureuse, insuffisante et des particules de cire ou de matière adhésive difficiles à enlever restent collées à la peau.

Par GB-A-2 079 762 il est connu un procédé d'épilation selon lequel on recouvre les zones à épiler par une composition liquide de polyuréthanne, on laisse réticuler la composition, et on retire par traction la composition réticulée et les poils. Un tel procédé n'est pas utilisable du fait de l'action corrosive et toxique des fonctions isocyanate sur la peau.

La présente invention a précisément pour but de proposer un procédé d'épilation à froid qui ne présentent pas les inconvénients des produits et des procédés connus, ou du moins que sous une forme très atténuée.

Elle a également pour but de proposer un procédé d'épilation efficace, économique et de mise en oeuvre aisée, sans chauffage et totalement dépourvue de toxicité vis à vis de la peau.

Ces buts et d'autres sont atteints par la présente invention qui concerne en effet un procédé d'épilation pour supprimer les poils de la zone de peau ou leur présence est considérée comme inopportune, caractérisé en ce qu'on recouvre la zone à épiler d'une couche d'une composition organopolysiloxane réticulable à température ambiante en un élastomère silicone, on laisse la couche réticuler et on enlève de la peau par traction la couche d'élastomère durcie avec les poils.

De façon tout à fait surprenante et inattendue, la présente invention a permis de mettre en évidence que les élastomères silicones provenant des compositions organopolysiloxaniques réticulables à température ambiance, adhérent si fortement aux poils, que la simple traction d'une pellicule d'élastomère englobant ces poils permet de les arracher.

Il est préférable de procéder, avant l'épilation à un dégraissage des poils, afin de favoriser leur adhérence sur l'élastomère silicone. On peut utiliser une solution aqueuse contenant un émulsionnant doux avec de l'alcool éthylique.

Selon une variante, on peut, avant l'épilation, imprégner ou revêtir un support souple d'une composition organopolysiloxane réticulable à température ambiante puis l'appliquer sur la peau. Puis, pour l'épilation après durcissement on tire rapidement par saccades le support souple.

Les compositions organopolysiloxanes présentent l'avantage d'être totalement inoffensives vis à vis de la peau.

Dans le cadre de l'invention en peut utiliser n'importe quelle composition polysiloxane réticulant à froid en présence de l'humidité de l'air et/ou d'un catalyseur de réticulation et présentée dans un seul emballage (composition monocomposante) ou dans deux emballages distincts (composition bicomposante), le contenu des deux emballages étant mélangé de façon extemporanée lors de l'emploi.

Le produit épilatoire utilisé dans le procédé de l'invention se présente de préférence à l'état liquide ou pâteux.

Ces compositions organopolysiloxanes réticulables (durcissables) à froid éventuellement sous forme d'une mousse sont amplement connues et décrites dans la littérature, en particulier dans l'ouvrage de Walter Noll "Chemistry and Technology of Silicones" Academic Press, 1968, 2ème édition, pages 395 à 399.

Ces compositions organopolysiloxanes comportent un polymère organopolysiloxane de base qui va des fluides (au moins sept motifs siloxanes par molécule) aux gommes ne fluant pas. Comme indiqué plus haut, tout système de réticulation peut être employé pour le siloxane.

Il est donc possible d'utiliser une grande variété de compositions monocomposantes ou bicomposantes réticulant par des réactions de polyaddition ou de polycondensation en présence d'un catalyseur métallige et éventuellement d'une amine et d'un agent de réticulation qui est généralement un silane porteur de groupes hydrolysables.

Les compositions organopolysiloxanes bicomposantes ou monocomposantes réticulant à température ambiante par des réactions de polyaddition essentiellement par réaction de groupes ≡ SiH portés par un silane ou un polyorganosiloxane sur des groupes hydrocarbonés à insaturation alcéniques le plus souvent vinyliques reliés à un atome de silicium d'un polyorganosiloxane en présence généralement d'un catalyseur métallique, de préférence au platine, sont en particulier décrites dans les brevets américains US-A-3 220 972, US-A-3 284 406, US-A-3 436 366, US-A-3 697 473 et US-A-4 340 709.

Ces compositions organopolysiloxane comportent généralement:

- un polymère diorganopolysiloxane comportant par molécule au moins deux radicaux vinyles liés au silicium et présentant une viscosité d'au moins 50 mPa.s à 20°C;
- un polymère diorganopolysiloxane comportant par molécule au moins 3 atomes d'hydrogène liés au silicium et présentant une viscosité d'au moins 50 mPa.s à 20°C;
- une charge;
- une quantité catalytiquement efficace d'un métal ou d'un complexe de métal, le métal étant de préférence le platine, ou un métal du groupe du platine.

Les compositions bicomposantes réticulant par des réactions de polycondensation sont les compositions préférées. Elles comportent généralement une huile alpha-oméga-dihydroxydiorganopolysiloxe, une charge et un agent de réticulation qui est un silane portant au moins trois groupements hydrolysables ou un polysiloxane provenant de l'hydrolyse partielle de ce silane, en présence d'une quantité catalytiquement efficace d'un catalyseur métallique et/ou d'une amine, on peut citer les compositions décrites dans les brevets américains US-A-3 678 002, US-A-3 888 815, US-A-3 933 729, GB-A-2 032 936 et le brevet français FR-11 179 962.

Parmi ces compositions élastomériques on préfère tout particulièrement celles pour lesquelles l'agent de réticulation est un silicate, un alkyltrialcoxysilane ou un polysilicate et pour lequel le catalyseur métallique est un sel d'étain et en particulier les compositions comportant:

1. Au moins un polymère alpha-oméga-dihydroxydiorganopolysiloxane de viscosité de 500 à 1 000 000 mPa.s à 25°C, dont les radicaux organiques sont des radicaux hydrocarbonés monovalents.
2. Au moins une charge.
3. Au moins un agent de réticulation choisi parmi:
- les polyalcoxysilanes de formule:

$$(R_1O)_a\ Si\ (R_2)_{4-a}\ (Ia)$$

dans laquelle a est 3 ou 4, $R_1$ et $R_2$, identiques ou différents choisis parmi un radical hydrocarboné monovalent ayant jusqu'à 8 atomes de carbone, $R_1$ peut signifier en outre un radical $R_3OR_1$ dans lequel $R_3$ est un radical hydrocarboné divalent ayant jusqu'à 6 atomes de carbone et $R_1$ a la signification ci-dessus; (Ia)

- les polyalcoxysiloxanes présentant au moins deux radicaux alcoxy liés à un atome de silicium par molécule dans lesquels les atomes de silicium sont reliés par des liaisons Si-O-Si, les autres valences des atomes de silicium étant satisfaites par des radicaux $R_1O$ ou $R_2$, $R_1$ et $R_2$ ayant la signification indiquée ci-dessus.

4. Une quantité catalytiquement efficace d'au moins un composé catalytique de l'étain.

Les polymères alpha-oméga-dihydroxydiorganopolysiloxanes (1), utilisés dans les compositions de l'invention ont une viscosité de 500 à 1 million de mPa.s à 25°C, de préférence 800 à 500 000 mPa.s à 25°C, ils sont constitués principalement de motifs diorganosiloxyles mais la présence d'autres motifs tels que monoorganosiloxyles n'est pas exclue dans la proportion d'au plus 2 % en nombre.

A titre de radicaux organiques, liés aux atomes de silicium de ces polymères, peuvent être cités ceux du type:

- alkyle ayant de 1 à 4 atomes de carbone comme les radicaux méthyle, éthyle, propyle;
- halogénoalcoyle ayant de 3 à 4 atomes de carbone comme les radicaux trifluoro-3,3,3 propyle, trifluoro-4,4,4 butyle;
- aryle ayant de 6 à 8 atomes de carbone comme les radicaux phényle, tolyle, xylyle;
- halogénoaryle ayant de 6 à 7 atomes de carbone comme les radicaux chlorophényle, dichlorophényle, trichlorophényle, tétrachlorophényle, trifluorométhylphényle;
- cyanoalkyle ayant de 3 à 4 atomes de carbone comme les radicaux béta-cyanoéthyle, gamma-cyanopropyle.

Les radicaux méthyles représentent au moins 60 % de la totalité de ces radicaux organiques, de préférence 75 %.

Les agents de réticulation (3) utilisés, comme mentionnés plus haut, à raison d'au plus 15 parties, de préférence d'au plus 7 parties, pour 100 parties de polymère (1) servent principalement à

réticuler la composition.

Les agents de réticulation (3) sont des produits bien connus décrits notamment dans les brevets français 1 330 625, 2 121 289, 2 121 631 et 2 458 572 cités comme référence.

Par radical hydrocarboné monovalent pour $R_1$ et $R_2$, on entend, plus particulièrement, des radicaux alkyle, alcényle, alcoxyalkyle, phényle, alkylphényle, phénylalkyle, éventuellement substitués par un atome d'halogène.

On peut par exemple utiliser les silanes de formule:

$CH_3Si(OCH_3)_3$
$CH_3Si(OCH_2CH_3)_3$
$CH_3Si(OCH_2CH_2OCH_3)_3$
$Si(OCH_2CH_2OCH_3)_4$
$Si(OCH_3)_4$
$Si(OCH_2CH_3)_4$
$Si(OCH_2CH_2CH_3)_4$
$CH_2 = CHSi(OCH_2CH_2OCH_3)_3$
$C_6H_5Si(OCH_3)_3$
$C_6H_5Si(OCH_2CH_2OCH_3)_3$
$CH_3Si(OCH_2\text{-}CHOCH_3)_3$
        |
        $CH_3$

Parmi les agents de réticulation (3) on préfère plus particulièrement les alkyltrialcoxysilanes, les silicates d'alkyle et les polysilicates d'alkyle dans lesquels les radicaux organiques sont des radicaux alkyles ayant de 1 à 4 atomes de carbone.

Les silicates d'alkyle peuvent être choisis parmi le silicate de méthyle, le silicate d'éthyle, le silicate d'isopropyle, le silicate de n-propyle, et les polysilicates choisis parmi les produits d'hydrolyse partielle de ces silicates ce sont des polymères constitués d'une proportion importante de motifs de formule $(R^4O)_2SiO$ et d'une faible proportion de motifs de formule $(R^4O)_3SiO_{0,5}$, $R^4OSiO_{1,5}$ et $SiO_2$; le symbole $R^4$ représentant les radicaux méthyle, éthyle, isopropyle, n-propyle. Pour les caractériser on se base habituellement sur leur teneur en silice qui est établie par dosage du produit d'hydrolyse totale d'un échantillon.

Leurs méthodes de préparation sont bien connues et figurent en particulier dans l'ouvrage "Chemistry and Technology of Silicones" de W. Noll aux pages 648 à 659. Ces polymères, afin d'être compatibles et/ou réactifs avec les autres ingrédients mis en oeuvre pour la préparation des compositions de l'invention, doivent posséder la faculté de se dissoudre dans les solvants hydrocarbonés usuels tels que le toluène, le xylène, le méthylcyclohexane dans la proportion d'au moins 50 parties de polymères pour 100 parties de solvants.

On peut en particulier utiliser comme polysilicate, un silicate d'éthyle partiellement hydrolysé commercialisé sous la marque "Ethyl Silicate-40" par Union Carbide Corporation, ou un silicate de propyle partiellement hydrolysé.

Le composé (4) est un composé catalytique à l'étain utilisé ou proposé pour catalyser ce type de composition, et qui peut être en particulier un sel d'étain d'un acide mono- ou dicarboxylique. Ces carboxylates d'étain sont décrits notamment dans l'ouvrage de Noll (Chemistry and Technology of Silicones, page 337, Academic Press, 1968, 2ème édition). On peut en particulier citer le naphténate, l'octanoate, l'oléate, le butyrate, le dilaurate de dibutylétain, le diacétate de dibutylétain.

On peut également utiliser les sels d'acides monocarboxyles ramifiés sur un atome de carbone aliphatique en position alpha par rapport au groupe carboxyle et ayant au moins 8 atomes de carbone par molécule tels que décrits dans le brevet français 2 066 159 en particulier le diversatate de dibutylétain.

On peut également utiliser comme composé catalytique à l'étain le produit de réaction d'un sel d'étain, en particulier d'un dicarboxylate d'étain sur du polysilicate d'éthyle comme décrit dans le brevet US-A-3 186 963. On peut également utiliser le produit de réaction d'un dialkyldialcoxysilane sur un carboxylate d'étain comme décrit dans le brevet US-A-3 862 919.

On peut également utiliser le produit de réaction d'un silicate d'alkyle ou d'un alkyle trialcoxysilane sur le diacétate de dibutylétain comme décrit dans le brevet belge 842 305.

Dans les compositions organopolysiloxanes on utilise comme charges généralement une silice de pyrogénation, une silice de précipitation, des terres de diatomées, du carbonate de calcum et du quarts broyé. Pour les compositions réticulant par polycondensation, les silices sont de préférence traitées par un chlorosilane, un siloxane ou disilazane tel que l'hexaméthyldisilazane et les compositions organopolysiloxaniques décrites dans les brevets américains US-A-4 064 096 US-A-3 642 685 et dans le brevet français FR-A-2 208 937 conviennent plus particulièrement.

Les compositions monocomposantes réticulant par des réactions de polycondensation à l'humidité de l'air, éventuellement en présence d'un catalyseur métallique (Ti, Sn), sont également utilisables et comportent généralement une huile alpha-oméga-dihydroxypolyorganosiloxanique ou une huile polyorganosiloxane bloquée à chaque extrémité par au moins deux groupes organiques hydrolysables tels que des groupes alcoxy ou acyloxy et un silane portant au moins trois groupements organiques hydrolysables; l'emploi de ce silane n'est pas indispensable si on utilise une huile bloquée.

Suivant la nature de ces groupements, les compositions monocomposantes sont dites acides, neutres ou basiques.

Comme compositions acides on peut utiliser par exemple les compositions décrites dans les brevets US-A-3 035 016, 3 077 465, 3 133 891, 3 409 573, 3 438 930, 3 647 917 et 3 886 118.

Comme compositions neutres, on peut utiliser par exemple les compositions décrites dans les brevets US-A-3 065 194, 3 542 901, 3 689 454, 3 779 986, GB-A-2 052 540, US-A-4 417 042 et EP-A-69 256.

Comme compositions basiques on peut par exemple utiliser les compositions décrites dans les brevets US-A-3 378 520, 3 364 160, 3 417 047, 3 464 951, 3 742 004 et 3 758 441.

Les compositions neutres sont préférées. On peut également utiliser les compositions coulantes monocomposantes telles que celles décrites dans les brevets US-A-3 922 246, 3 965 280 et 4 143 088.

On peut également utiliser des compositions organopolysiloxaniques réticulant sous forme d'une mousse élastomérique.

Des compositions de ce type convenant plus particulièrement sont décrites dans le brevet américain US-A-3 070-555.

Elles comportent de préférence:

1) un polymère alpha-oméga-dihydroxydiorganopolysiloxane de viscosité d'au moins 50 mPa.s à 25°C;
2) un polymère diorganopolysiloxane présentant de 1 à 75 % en poids de motifs siloxanique présentant un atome d'hydrogène lié directement à un atome de silicium;
3) jusqu'à 50 % en poids basé sur le poids du polymère 1) d'au moins un composé choisi parmi des silanols, des siloxanes hydroxylés de bas poids moléculaire, d'eau et d'alcools;
4) un catalyseur à l'étain.

Le catalyseur à l'étain est de préférence l'octoate stanneux.

Ces compositions peuvent être conditionnées en aérosol, comme décrit dans le brevet européen EP-A-8 034 et dans les demandes de brevet allemand DE-A-2 909 443 et DE-A-2 911 971.

Elles peuvent contenir jusqu'à 30 % en poids d'une charge choisie parmi de la silice de pyrogénation ou de précipitation, des terres de diatomées, du quarts broyé, des argiles et des oxydes de fer.

Dans les polymères ou les silanes 1), 2) et 3) ci-dessus, les radicaux organiques sont analogues à ceux décrits ci-dessus pour les polymères des compositions bicomposantes récitulant par des réactions de polycondensation. Les radicaux méthyle et phényle sont toutefois préférés.

En outre, la composition réticulant sous forme de mousse peut comporter une charge usuelle (silice, terre de diatomées, quarts broyé etc...).

Les exemples suivants illustent l'invention, dans tout ce qui suit les pourcentages sont exprimés en poids:

**Exemple 1**

Dans un malaxeur de 18 litres, équipé de bras de malaxage à pales pleines, maintenu sous atmosphère inerte par le passage d'un léger courant d'azote, sont successivement introduits (les bras de malaxage étant en marche):

- 3000 g d'une huile diméthylpolysiloxane de viscosité 1000 mPa.s à 25°C, terminée à chaque extrémité de sa chaîne par un groupe de formule $(CH_3)_3SiO_{0,5}$;
- 1200 g d'une silice de combustion de surface spécifique 200 $m^2/g$ ayant 1.5 % d'eau adsorbée;
- 96 g d'eau distillée;
- 240 g d'hexaméthyldisilazane.

Ce mélange est brassé pendant 6 heures à la température ambiance, il est ensuite débarrassé de ses composants volatils par chauffage de sa masse vers 155°C pendant 6 heures. Pendant toute la durée de chauffage un courant d'azote circule dans le malaxeur au débit de 200 l/h.

A ce mélange refroidi vers 80°C sont successivement ajoutés:

- 3000 g d'un quarts broyé de diamètre particulaire moyen 5 µm, de surface spécifique 15 $m^2/g$ environ ayant 1 % d'eau adsorbée;
- 3000 g d'une huile alpha-oméga-dihydroxydiméthylpolysiloxane de viscosité 16 000 mPa.s à 25°C;
- 52 g d'une huile alpha-oméga-dihydroxydiméthylpolysiloxanique de viscosité 50 mPa.s à 25°C.

L'ensemble est malaxé pendant 2 heures, la composition organopolysiloxane formée est ensuite soumise à un broyage énergique par passage sur un broyeur 3 cylindres à serrage hydraulique, chaque cylindre ayant un diamètre de 120 mm et les pressions de serrage étant de 10 et 20 $kg/cm^2$.

La composition A obtenue est conservée à l'abri de l'air.

100 g de la composition A sont mélangés avec 4,7 g d'un système de prise B constitué de:

- 3,5 g (comme diluant) d'une diméthylpolysiloxane de viscosité 20 mPa.s à 25°C, bloquée à chaque extrémité de sa chaine par un groupe de formule $(CH_3)_3SiO_{0,5}$;
- 0,7 g (comme réticulant organosilicique) d'un polysilicate de n-propyle titrant 34 % de silice;
- 0,5 g (comme catalyseur métallique) de dilaurate de dibutylétain.

Le mélange de A et B a lieu par simple agitation.

Cette composition est étalée, en une couche de 2 mm d'épaisseur environ, sur un mollet à épiler dont les poils ont été préalablement dégraissés par une solution alcoolique. Au bout de 20 minutes, la couche d'élastomère réticulée est enlevée par coup sec et le mollet se trouve épilé.

**Exemple 2**

Dans le dispositif utilisé à l'exemple 1 sont successivement introduits:

- 3000 g d'une huile diméthylpolysiloxane de viscosité 1800 mPa.s à 25°C, terminée à chaque extrémité de sa chaîne par un groupe de formule $(CH_3)_2CH_2 = CHSiO_{0,5}$;
- 1500 g d'une silice de combustion de surface spécifique 300 $m^2/g$ ayant 1,9 % d'eau adsorbée;
- 140 g d'eau distillée;
- 260 g du disilazane de formule $(CH_3)_3SiNH$-$Si(CH_3)_2CH{=}CH_2$.

Le mélange est brassé 7 heures à la température ambiante, ensuite il est porté vers 165°C et maintenu à cette température pendant 5 heures. Au cours de ce chauffage un courant d'azote circule dans le malaxeur au débit de 250 l/h afin d'entraîner tous les produits volatils.
Dans ce mélange, refroidi vers 70°C, sont successivement introduits:

- 2500 g d'un quartz broyé, de diamètre particulaire moyen 7 µm, de surface spécifique 10 $m^2/g$ environ ayant 1,3 % d'eau adsorbée;
- 2600 g d'une huile alpha-oméga-dihydroxydiméthylpolysiloxane de viscosité 25 000 mPa.s à 25°C;
- 80 g d'une huile alpha-oméga-dihydroxydiméthylpolysiloxane de viscosité 20 mPa.s à 25°C.

L'ensemble est malaxé pendant 3 heures à la température ambiante, la composition organopolysiloxanique obtenue est alors soumise à un broyage par passage sur le broyeur décrit à l'exemple 1. La composition A obtenue est conservée à l'abri de l'air.

1000 g de la composition A sont mélangés avec 22 g d'un système de prise B constitué de:

- 12 g (comme diluant) d'une huile diméthylpolysiloxane de viscosité 20 mPa.s a 25°C, bloquée à chaque extrémité de sa chaîne par un groupe de formule $(CH_3)_3SiO_{0,5}$;
- 4 g (comme réticulant organosilicique) d'orthosilicate de propyle;
- 4 g (comme catalyseur métallique) de diacétate de dibutylétain.

Dans le mélange de A et B on imprègne un tissu de coton que l'on applique sur un mollet à épiler. Au bout de 15 minutes, après durcissement de l'élastomère on retire par petit coup sec le tissu et le mollet se trouve correctement épilé.

**Exemple 3**

On prépare une première composante A résultant du mélange de:

- 630 g d'une huile alpha-oméga-dihydroxydiméthylpolysiloxane de viscosité 3 500 mPa.s à 25°C;
- 360 g d'une huile alpha-oméga-dihydroxydiméthylpolysiloxane de viscosité 25 mPa.s à 25°C;
- 60 g d'une huile méthylhydrogénopolysiloxane bloquée à chacune de ses extrémités par un groupe triméthylsiloxy et de viscosité 23 mPa.s à 25°C;
- 30 g d'orthosilicate de n-propyle;
- 60 g de diphénylméthylsilanol;
- 160 g de quartz finement broyé.

A 100 g de composante A on incorpore par malaxage 3 g d'octoate stanneux.

On étale le mélange sur un mollet à épiler. Le moussage intervient dès l'incorporation du catalyseur. La mousse est à l'état d'élastomère en 10 - 15 minutes et son arrachement dégage le mollet convenablement épilé.

**Revendications**

1. Procédé d'épilation pour supprimer les poils de la zone de peau où leur présence est considérée comme inopportune, caractérisé en ce qu'on recouvre la zone à épiler d'une couche d'une composition organopolysiloxane réticulable à température ambiante en un élastomère silicone, on laisse la couche réticuler et on enlève de la peau par traction la couche d'élastomère durcie avec les poils.

2. Procédé selon la revendication 1, caractérisé en ce que la composition organopolysiloxane est choisie parmi les compositions monocomposantes ou bicomposantes réticulant par des réactions de polyaddition ou de polycondensation en présence d'un catalyseur métallique.

3. Procédé selon la revendication 1 ou 2, caractérisé en ce que la composition organopolysiloxane comporte:

- un polymère diorganopolysiloxane comportant par molécule au moins deux radicaux vinyles liés au silicium et présentant une viscosité d'au moins 50 mPa.s à 20°C,
- un polymère diorganopolysiloxane comportant par molécule au moins 3 atomes d'hydrogène liés au silicium et présentant une viscosité d'au moins 50 mPa.s à 20°C,
- une charge,
- une quantité catalytiquement efficace d'un métal ou d'un complexe de métal, le métal étant de préférence le platine, ou un métal du groupe du platine.

4. Procédé selon la revendication 1 ou 2, caractérisé en ce que la composition organopolysiloxane comporte une huile alpha-oméga-dihydroxydiorganopolysiloxane, une charge, un agent de réticulation choisi parmi un silane portant au moins trois groupements hydrolysables et un polysiloxane provenant de l'hydrolyse partielle de ce silane et d'une quantité catalytiquement efficace d'un catalyseur métallique et/ou d'une amine.

5. Procédé selon la revendication 4, caractérisé en ce que la composition se présente sous forme

bicomposante et en ce que le catalyseur est un sel d'étain.

6. Procédé selon la revendication 4 ou 5, caractérisé en ce que la charge est traitée par un chlorosilane, un siloxane ou un disilazane.

7. Procédé selon la revendication 1 ou 2, caractérisé en ce que la composition organopolysiloxane réticule sous forme d'une mousse élastomérique.

8. Procédé selon la revendication 7, caractérisé en ce que la composition organopolysiloxane réticulant sous forme d'une mousse comporte:

a) - un polymère alpha-oméga-dihydroxydiorganopolysiloxane de viscosité d'au moins 50 mPa.s à 25°C,
b) - un polymère diorganopolysiloxane présentant de 1 à 75 % en poids de motifs siloxane présentant un atome d'hydrogène lié directement à un atome de silicium,
c) - jusqu'à 50 % en poids basé sur le poids du polymère a) d'au moins un composé choisi parmi des silanols, des siloxanes hydroxylés de bas poids moléculaire, d'eau et d'alcools,
d) - un catalyseur à l'étain.

**Patentansprüche**

1. Epilationsverfahren zur Beseitigung der Haare aus der Hautzone, wo ihre Anwesenheit als unangebracht angesehen wird, dadurch gekennzeichnet, daß man die zu epilierende Zone mit einer Schicht einer Organopolysiloxanzusammensetzung bedeckt, die bei Umgebungstemperatur zu einem Silikonelastomeren vernetzbar ist, daß man die Schicht vernetzen läßt und die gehärtete Elastomerschicht mit den Haaren von der Haut durch Zug entfernt.

2. Verfahren gemäß Anspruch 1, dadurch gekennzeichnet, daß die Organopolysiloxanzusammensetzung ausgewählt ist unter den Einkomponenten- oder Bikomponentenzusammensetzungen, die durch Polyadditions- oder Polykondensationsreaktionen in Gegenwart eines Metallkatalysators vernetzen.

3. Verfahren gemäß Anspruch 1 oder 2, dadurch gekennzeichnet, daß die Organopolysiloxanzusammensetzung enthält:

- ein Diorganopolysiloxanpolymeres das pro Molekül mindestens zwei Vinylreste, gebunden an das Silicium, trägt und eine Viskosität von mindestens 50 mPa.s bei 20°C aufweist,
- ein Diorganopolysiloxanpolymeres, das pro Molekül mindestens drei Wasserstoffatome, gebunden an das Silicium, enthält und eine Viskosität von mindestens 50 mPa.s bei 20°C aufweist,
- einen Füllstoff,
- eine katalytisch wirksame Menge eines Metalls oder Metallkomplexes, wobei das Metall vorzugsweise Platin oder ein Metall aus der Platingruppe ist.

4. Verfahren gemäß Anspruch 1 oder 2, dadurch gekennzeichnet, daß die Organopolysiloxanzusammensetzung enthält ein alpha-omega-Dihydroxydiorganopolysiloxanöl einen Füllstoff, ein Vernetzungsmittel, ausgewählt unter einem Silan mit mindestens drei hydrolysierbaren Gruppen und einem Polysiloxan, stammend von der partiellen Hydrolyse dieses Silans und aus einer katalytisch wirksamen Menge eines Metall- und/oder Aminkatalysators.

5. Verfahren gemäß Anspruch 4, dadurch gekennzeichnet, daß die Zusammensetzung in Bikomponentenform vorliegt und daß der Katalysator ein Zinnsalz ist.

6. Verfahren gemäß Anspruch 4 oder 5, dadurch gekennzeichnet, daß der Füllstoff mit einem Chlorsilan, einem Siloxan oder einem Disilazan behandelt ist.

7. Verfahren gemäß Anspruch 1 oder 2, dadurch gekennzeichnet, daß die Organopolysiloxanzusammensetzung in Form eines elastomeren Schaums vernetzt.

8. Verfahren gemäß Anspruch 7, dadurch gekennzeichnet, daß die in Form eines Schaums vernetzende Organopolysiloxanzusammensetzung enthält:

a) - ein alpha-omega-Dihydroxydiorganopolysiloxanpolymeres der Viskosität von mindestens 50 mPa.s bei 25°C,
b) - ein Diorganopolysiloxanpolymeres, das 1 bis 75 Gew.-% Siloxangruppierungen mit einem Wasserstoffatom, direkt gebunden an ein Siliciumatom, aufweist,
c) - bis zu 50 Gew.-%, bezogen auf das Gewicht des Polymeren a), mindestens einer Verbindung, ausgewählt unter den Silanolen, den hydroxylierten Siloxanen mit niedrigem Molekulargewicht, Wasser und Alkoholen,
d) - einen Katalysator mit Zinn.

**Claims**

1. Depilatory process for the removal of hairs from the zone of the skin where their presence is considered inopportune, characterized in that the zone to be depilated is covered with a layer of a composition of an organopolysiloxane capable of being cross-linked at ambient temperature in a silicone elastomer, the layer is left to cross-link, and the layer of hardened elastomer is removed from the skin together with the hairs by traction.

2. Process according to claim 1, characterized in that the organopolysiloxane composition is chosen from the monocomponent or bicomponent compositions which cross-link by polyaddition or polycondensation reactions in the presence of a metallic catalyst.

3. Process according to claim 1 or 2, characterized in that the organopolysiloxane composition comprises:

- a diorganopolysiloxane polymer containing, per molecule, at least two vinyl radicals bonded to the silicon, and having a viscosity of at least 50 mPa.s at 20°C,
- a diorganopolysiloxane polymer containing, per molecule, at least 3 hydrogen atoms bonded to the silicon, and having a viscosity of at least 50 mPa.s at 20°C,
- a filler,
- a catalytically effective quantity of a metal or of a metal complex, the metal preferably being platinum or a metal of the platinum group.

4. Process according to claim 1 or 2, characterized in that the organopolysiloxane composition comprises an α-Ω-dihydroxydiorganopolysiloxane oil, a filler, a cross-linking agent chosen from a silane having at least three hydrolysable groups and a polysiloxane resulting from the partial hydrolysis of this silane, and a catalytically effective quantity of a metallic catalyst and/or of an amine.

5. Process according to claim 4, characterized in that the composition is presented in a bicomponent form, and in that the catalyst is a tin salt.

6. Process according to claim 4 or 5, characterized in that the filler is treated with a chlorosilane, a siloxane or a disilazane.

7. Process according to claim 1 or 2, characterized in that the organopolysiloxane composition cross-links in the form of an elastomeric foam.

8. Process according to claim 7, characterized in that the organopolysiloxane composition which cross-links in the form of a foam comprises:

a) - an α-Ω-dihydroxydiorganopolysiloxane polymer with a viscosity of at least 50 mPa.s at 25°C,
b) - a diorganopolysiloxane polymer having from 1 to 75 % by weight of siloxane residues having a hydrogen atom directly bonded to a silicon atom,
c) - up to 50 % by weight, based on the weight of the polymer a), of at least one compound chosen from silanols, low molecular weight hydroxylated siloxanes, water and alcohols,

d) - a tin-containing catalyst.